Europäisches Patentamt

European Patent Office

Office européen des brevets

(19)

(11) Publication number: 0 328 816
A2

# EUROPEAN PATENT APPLICATION

(12)

(21) Application number: 88310817.7

(51) Int. Cl.4: **A61K 7/13**

(22) Date of filing: **16.11.88**

(30) Priority: **01.02.88 US 150676**

(43) Date of publication of application:
**23.08.89 Bulletin 89/34**

(84) Designated Contracting States:
**BE CH DE ES FR GB GR IT LI NL SE**

(71) Applicant: **Bristol-Myers Company**
**345 Park Avenue**
**New York New York 10154(US)**

(72) Inventor: **Brown, Keith**
**59 Douglas Road**
**New Canaan Connecticut 06840(US)**
Inventor: **Iscowitz, Sigmund**
**150-25 78th Avenue**
**Flushing New York 11367(US)**
Inventor: **Masoni, Jack**
**34 Ridgewood Drive**
**Betheny Connecticut 06525(US)**
Inventor: **Wolfram, Leszek**
**666 Westover Road**
**Stamford Connecticut 06902(US)**
Inventor: **Mayer, Alice**
**37 Jupiter Road**
**Bethel Connecticut 06801(US)**

(74) Representative: **Baillie, Iain Cameron et al**
**c/o Ladas & Parry Isartorplatz 5**
**D-8000 München 2(DE)**

(54) **Process for dyeing of waved or relaxed hair.**

(57) A process for chemically treating a head of human hair to straighten or wave said hair followed in close proximity by a metal ion catalyzed dyeing step to color said hair without substantial irritation to the scalp.

EP 0 328 816 A2

## PROCESS FOR DYEING OF WAVED OR RELAXED HAIR

### FIELD OF THE INVENTION

This invention relates to the field of hair waving or relaxing processes employing as an additional component catalyzed hair dyeing compositions, which processes are substantially non-irritating to the human scalp.

### BACKGROUND OF THE INVENTION

The use of various metal salts to catalyze oxidative reactions is well known. For example Fentons Reagent uses a ferrous ion and hydrogen peroxide as a powerful, and widely accepted oxidant.

It is, therefore, not unexpected that metal ions would increase the rate of oxidative coupling reactions such as those used in permanent hair dyeing. See for example German Patent 2,028,818 and French patent 2,045,991.

It is also known that metal ions increase the rate of auto-oxidative reactions (i.e. air oxidation). One system, which is taught in US Patent #4,004,877, uses a variety of metal ions for this purpose.

Thus, the prior art suggests that a useful increase in dyeing rate can be realized using metal ion catalysis for the type of reactions normally carried out in commercial hair dyeing procedures.

However, no commercial system utilizing this effect is known to the applicants. It is hypothesized that until now there has existed no motivation to utilize a catalyzed reaction for hair dyeing since no advantage other than pure speed could be appreciated.

Accepted and conventional wisdom dictates that hair dyeing should not be carried out in conjunction with hair waving or hair relaxing treatments. Contrary to this, applicants have found that the advantages appreciated through use of metal ion catalysis of hair dyeing systems allow one to perform a metal ion catalyzed dyeing procedure on just treated head of human hair, (e.g. hair which has been chemically straightened or waved). Moreover this can be accomplished without the irritation and soreness that is normally expected to occur under such circumstances.

A recent patent, US 4,630,621, attempts to overcome these problems by incorporating the oxidative dyeing and permanent wave neutralizing steps into a single process to be used after reductive waving. In the practice of this invention, the oxidative dye must be added to the permanent wave neutralizer immediately prior to use, since they are not compatible for even short term storage. While the inventor claims an adequate dyeout from this process in 3-5 minutes, in our hands these times produced a weaker than normal dyeout different from the shade and intensity desired by the consumer, and quite different from that described on the commercial dye product . Times in the order of 15-30 minutes are required to achieve the normal dyeout intensity and shade and these times are sufficient to cause extreme user discomfort and significant hair damage. In contrast, the current invention allows for development of the expected dye intensity and shade in dyeing times of 1-3 minutes with essentially no user discomfort or hair damage. In addition, the method described in the Pontani patent cannot be applied to sequentially dyeing chemically straightened hair, since this method does not involve an oxidative neutralization step.

### SUMMARY OF THE INVENTION

The present invention is directed to a process for sequentially chemically treating a head of human hair to straighten or wave the hair followed by, in substantially close proximity, the dyeing of the hair using a metal ion catalyzed oxidative hair dyeing composition.

### DESCRIPTION OF THE INVENTION

It has been found that the considerable advantages of increased dyeing rate to be achieved by utilizing metal ion catalysis of oxidative hair dyeing compositions are primarily useful in areas where more than one cosmetic treatment is to be performed, e.g. straightening or permanent waving in combination with dyeing. .These treatments are conventionally carried out separately from dyeing due to the increased possibility of irritation from the dye formulations on an already sensitized scalp. The dyeing step cannot be carried out before the other treatment step since most hair dyes are reactive to the chemicals used in relaxers and waving products.

Surprisingly, it has been found in actual head testing that there is no increased irritation when the hair is colored with a metal ion catalyzed system immediately after relaxing or waving. The added time required to sufficiently dye the hair is minimal since the increased porosity of the hair and its ability to bind more metal immediately after such waving or relaxing treatment make it conducive to rapid dyetake. In fact, it has been found that when dyeing treated hair, the catalyst concentration necessary to dye virgin hair can be reduced at least 10-fold. With the correct choice of catalyst, there is no apparent increase in the degree of hair damage.

The broad applicability of the process of the present invention is supported by the fact that all commercial types of hair relaxers or permanent waving systems can be used with relatively equal advantage. All commercial types of permanent, oxidative dyes can be used.

The following metal ions have been found to be useful to catalyze various oxidative dyes and are effective to various degrees:

Copper (II), Tin (II), Tin (IV), Antimony (III), Iron (II), Silver (I), Nickel (II), Lead (II), Zinc (11), Chromium, Cobalt, Magnesium, Manganese (II), Aluminum (III), and Titanium (II)

In addition, mixtures of the aforementioned metal ions can also be used to advantage.

Aqueous solutions of soluble salts (acetate, lactate, nitrate, chloride, etc.) are effective however, for cosmetic reasons, it is preferred to have surfactants also present. A fully formulated shampoo is a preferred vehicle for introducing the metal ion catalyst component to the surface of the hair.

The metal ion is applied after the relaxing or waving step and can be incorporated in most cases, if desired, into the neutralizing system used for these products. The initial application will preferentially be followed by a rinse to remove unbound metal ions.

The dye to be used is preferentially mixed with peroxide and applied for approximately 5 minutes to the hair. After rinsing off excess dye, the hair can be conditioned as required.

It has been found that in order to be effective the metal ion catalyst must be replenished for each subsequent hair treatment cycle.

It has also been found that the concentration of hydrogen peroxide used in the dyeing solution can be reduced significantly from the normal 6% solution which is conventionally employed in commercial hair dyeing systems. It is anticipated that a lower limit of about 1% hydrogen peroxide would still be effective.

## EXAMPLES

While the invention has been described above, the details of the present invention will be better understood by recourse to the following examples:

## EXAMPLE I

A sample of human hair is chemically straightened using Revlon Realistic brand of commercial relaxer, then rinsed and towel dried. A catalyst containing shampoo having the following formulation (CTFA-approved names are used).

EP 0 328 816 A2

| wt./wt. | |
|---|---|
| 0.25% | Manganese chloride |
| 10.00% | Disodium Cocamido MIPA Sulfo-Succinate |
| 0.10% | Methylparaben |
| 0.0008% | D & C Violet #2 |
| 0.0009% | FD & C Blue #1 |
| 40.4883% | Water |
| 1.0% | PPG-9 diethylammonium chloride |
| 0.20% | Imidazolidinyl urea |
| 0.05% | EDTA |
| 0.20% | Citric acid |
| 0.60% | Sodium chloride |
| 4.50% | Isostearoamphopropionate |
| 2.0% | Quaternium-22 |
| 40.0% | Ammonium lauryl sulfate |
| 0.40 | Hydrolyzed animal collagen |
| 0.01 | Laureth 23 |
| 0.20% | Fragrance |
| 100.00% | |

is applied to the hair for (1-5) minutes. The hair is then rinsed and towel dried. A commercial oxidation dye product of appropriate shade, mixed as directed, is applied to the hair and allowed to sit for 5 minutes. The hair is again rinsed and towel dried.

The hair which was originally curly and about 50% gray is now straight and uniformly colored (to the desired shade). The complete process takes about 40 minutes.

In the following examples the same procedure as in Example I, with the indicated variations in relaxer, catalyst composition and commercial dyeing system, was utilized with the same high degree of success.

### EXAMPLE II

Hair Type:     Ethnic untreated dark brown with 20% gray.
Relaxer:     Revlon® Realistic Relaxer - Regular

| 2.87% | $ZnSO_4.7\ H_2O$ (0.1 M) |
|---|---|
| 97.13% | Water |
| 100.00% | |

Dye:     Nice 'n Easy® #111 (Natural Tawny Auburn)
Result:     Straight dark brown with auburn highlights

### EXAMPLE III

Hair type:     Ethnic Red dyed hair with gray untreated regrowth
Relaxer:     Revlon® Realistic Relaxer - Mild
Catalyst:

| 2.03% | $MgCl_2.6\ H_2O$ (0.1 M) |
|---|---|
| 97.97% | Water |
| 100.00% | |

4

Dye:    Miss Clairol® 56 R (Cinnamon)
Result:    Straight Dark Reddish Brown hair


## EXAMPLE IV


Hair type:    Ethnic untreated salt & pepper hair
Relaxer:    Alberto-Culver® Regular No Lye Hair Relaxer
CATALYST:

| | |
|---|---|
| 1.44% | $ZnSO_4.7 H_2O$ (0.05 M) |
| 1.02% | $MgCl_2.6 H_2O$ (0.05 M) |
| 97.54% | Water |
| 100.00% | |

Dye:    Nice 'n Easy® #122 (Black)
Result:    Straight Black hair


## EXAMPLE V


Hair type:    Ethnic untreated salt & pepper hair
Relaxer:    Summit® Creme Relaxer - Regular
CATALYST:

| | |
|---|---|
| 2.87% | $ZnSO_4.7 H_2O$ (0.1 M) |
| 1.0% | SLES |
| 0.1% | Methylparaben |
| 0.2% | 2-phenoxyethanol |
| 95.83% | Water |
| 100.00% | |

Dye:    Miss Clairol® Creme Formula #52 D (Black Azure)
Result:    Straight Black hair


## EXAMPLE VI


Hair type:    Naturally curly medium brown hair with 50% gray
Relaxer:    Summit® Creme Relaxer - Mild
CATALYST:

| | |
|---|---|
| 0.28% | $FeSO_4.7 H_2O$ (0.01 M) |
| 0.25% | $CuSO_4.5 H_2O$ (0.01 M) |
| 99.47% | Water |
| 100.00% | |

Dye:    Nice 'n Easy® #118 (Natural Medium Brown)
Result:    Straight medium brown hair


## EXAMPLE VII

Hair type:   Straight light brown hair with 20% gray
Perm:   Clairol® Kind to Hair® for Normal Hair
CATALYST:   Same as Example II
Dye:   Same as Example II
Result:   Curly Tawny Auburn hair


## EXAMPLE VIII


Hair type:   Straight dyed red hair
Perm:   Helene Curtis® Quantum Extra Body Formula Acid perm
CATALYST:   Same as Example III
Dye:   Same as Example III
Result:   Curly Dark Reddish Brown hair


## EXAMPLES IX


Hair type:   Straight black salt & pepper hair
Perm:   Clairol Kindness® Extra Curly Perm for Natural Hair
CATALYST:   Same as Example IV
Dye:   Same as Example IV
Result:   Curly Black hair

While the invention has been described with respect to various specific examples and embodiments, it is to be understood that the invention is not limited thereto and that it can be variously practiced within the scope of the following claims.


**Claims**

1. A process for straightening or waving a head of human hair which comprises chemically treating said human hair, and substantially immediately thereafter dyeing said hair using a metal ion catalyzed oxidative hair dyeing composition.

2. A process for sequentially chemically treating and dyeing a head of human hair according to claim 1 comprising:
contacting said hair with a chemical waving or straightening agent to effect straightening or waving of said hair,
rinsing and towel drying said contacted hair,
applying to said rinsed and dried hair a surfactant formulation comprising an effective amount of one or more metal ion containing salts,
rinsing and towel drying said hair,
applying a commercial oxidative dyeing system to said hair,
and finally rinsing and towel drying said hair,
all of the above steps being accomplished without substantial time elapsing between steps and without excessive irritation of the scalp.

3. The process of claim 1, wherein the metal ions used to catalyze the oxidative hair dyeing composition are selected from the group consisting of:
Copper (II), Tin (II), Tin (IV), Antimony (III), Iron (II), Silver (I), Nickel (II), Lead (II), Zinc (II), Chromium, Cobalt, Magnesium, Manganese (II), Aluminum (III), and Titanium (II).

4. The process of claim 1, wherein the metal ions used to catalyze the oxidative dyeing composition are present as one or more acetate, lactate, nitrate or chloride salts.

5. The process of claim 1, wherein the metal ions used to catalyze the oxidative dyeing composition are applied as a component of a surfactant containing formulation.

6

6. The process of claim 1, wherein the metal ions used to catalyze the oxidative dyeing composition are applied as a component of a fully formulated shampoo composition.

7. The process of claim 1, wherein the metal ions used to catalyze the oxidative dyeing composition are incorporated into the neutralizing system used in conjunction with the commercial straightening or waving products first applied.

8. The process of claim 1, wherein the oxidative hair dyeing composition applied contains from about 1% to about 6% hydrogen peroxide.

9. The process of claim 1, wherein the oxidative hair dyeing composition applied contains about 1% hydrogen peroxide.